(19) Europäisches Patentamt
European Patent Office
Office européen des brevets

(11) EP 0 973 734 B1

(12) FASCICULE DE BREVET EUROPEEN

(45) Date de publication et mention de la délivrance du brevet:
**04.06.2003 Bulletin 2003/23**

(21) Numéro de dépôt: **99903551.2**

(22) Date de dépôt: **29.01.1999**

(51) Int Cl.[7]: **C07C 311/48**, C07C 317/00, C07C 317/18, C07C 317/22, C07D 317/42, C08F 14/18, C08F 16/32, C25B 9/00, H01M 8/10, C07B 61/00

(86) Numéro de dépôt international:
**PCT/CA99/00083**

(87) Numéro de publication internationale:
**WO 99/038842 (05.08.1999 Gazette 1999/31)**

# (54) DERIVES BIS-SULFONYLES POLYMERISABLES ET LEUR UTILISATION DANS LA PREPARATION DE MEMBRANES ECHANGEUSES D'IONS

POLYMERISIERBARE BIS-SULFONYLDERIVATE UND IHRE VERWENDUNG IN DER HERSTELLUNG VON IONENAUSTAUSCHERMEMBRANEN

CROSSLINKABLE BI-SULPHONYL DERIVATIVES AND THEIR USES FOR PREPARING ION-EXCHANGING MEMBRANES

(84) Etats contractants désignés:
**DE FR GB IT**

(30) Priorité: **30.01.1998 CA 2228466**
**28.04.1998 CA 2236196**

(43) Date de publication de la demande:
**26.01.2000 Bulletin 2000/04**

(60) Demande divisionnaire:
**03004041.4 / 1 312 603**

(73) Titulaire: **HYDRO-QUEBEC**
**Montréal Québec H2Z 1A4 (CA)**

(72) Inventeurs:
- **MICHOT, Christophe**
  **F-3800 Grenoble (FR)**
- **ARMAND, Michel**
  **Montréal, Québec H3T 1N2 (CA)**

(74) Mandataire: **Guerre, Dominique et al**
**Cabinet Germain et Maureau,**
**12, rue Boileau,**
**BP 6153**
**69466 Lyon Cedex 06 (FR)**

(56) Documents cités:
**WO-A-95/26056**
**WO-A-98/50349**
**WO-A-99/05126**
**US-A- 3 676 143**
**US-A- 5 627 292**

- **DESMARTEAU D D: "Novel perfluorinated ionomers and ionenes" JOURNAL OF FLUORINE CHEMISTRY, vol. 72, no. 2, 1 juin 1995, pages 203-208, XP002025806**
- **HIRAI K ET AL: "The double cycloaddition of disulfene and its related reactions" BULLETIN OF THE CHEMICAL SOCIETY OF JAPAN, vol. 43, no. 2, février 1970, pages 488-91, XP002103383**
- **CHEMICAL ABSTRACTS, vol. 60, no. 3, 3 février 1964 Columbus, Ohio, US; abstract no. 2771a, XP002103384 & JP 63 013056 A (NITTO CHEMICAL INDUSTRY CO., LTD. ET AL)**
- **DATABASE CROSSFIRE Beilstein Institut für Literatur der organischen Chemie XP002103385 & RUSS. J. ORG. CHEM., vol. 31, no. 4, 1995, pages 520-4,**
- **DATABASE CROSSFIRE Beistein Institut für Literatur der organischen Chemie XP002103386 & ORG. PREP. PROCED. INT., vol. 28, no. 6, 1996, pages 683-90,**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Jouve, 75001 PARIS (FR)

## Description

La présente invention concerne les résines échangeuses d'ions de type cationique, en particulier sous forme de membranes, éventuellement perfluorées, utiles en particulier dans les applications électrochimiques comme les piles à combustibles, les procédés chlore-soude, l'électrodialyse, la production d'ozone, les capteurs etc, ou tout autre application liée à la dissociation des centres anioniques fixés sur la membrane, tels que la catalyse hétérogène en chimie organique.

## ART ANTÉRIEUR

À cause de leur inertie chimique, les membranes échangeuses d'ions partiellement ou totalement fluorées sont habituellement choisies dans des procédés chlore-soude ou des piles à combustible consommant de l'hydrogène ou du méthanol. De telles membranes sont disponibles commercialement sous des appellations telles que Nafion$^{TM}$, Flemion$^{TM}$, DOW$^{TM}$. D'autres membranes similaires sont proposées par Ballard Inc. dans la demande WO 97/25369, qui décrit des copolymères de tétrafluoroéthylène et de perfluorovinyléthers ou trifluorovinylstyrène. Les monomères actifs à partir desquels ces copolymères sont obtenus portent des fonctions chimiques qui sont des précurseurs de groupements ioniques de type sulfonate ou carboxylate. Des exemples de ces précurseurs sont:

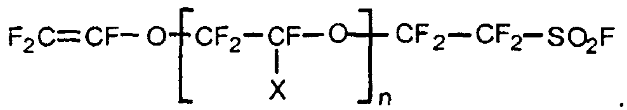

;

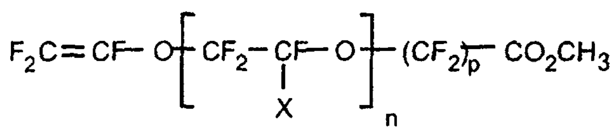

ou

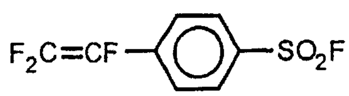

,

dans lesquels

- X est F, Cl or $CF_3$ ;
- n est 0 à 10 inclusivement; et
- p est 1 ou 2 ;

Les polymères aromatiques de type polyimide ou polyéther sulfones sulfonés ont aussi été envisagés, par exemple

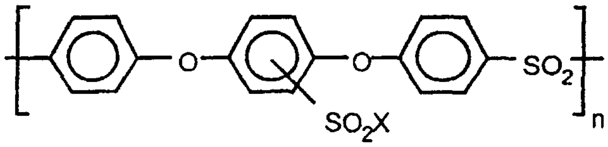

ou

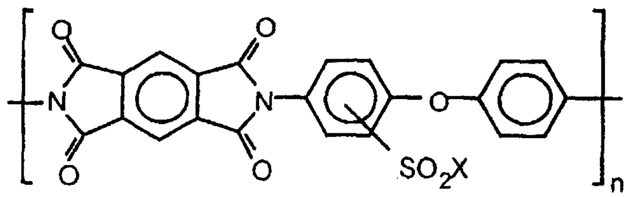

Une fois obtenu, le copolymère contenant les précurseurs précités est moulé, par exemple sous forme de feuilles, puis converti sous forme ionique par hydrolyse pour donner des espèces de type sulfonate ou carboxylate. Le cation associé aux anions sulfonate et carboxylate inclut le proton, le cation d'un métal alcalin ($Li^+$, $Na^+$, $K^+$); le cation d'un métal alcalino-terreux ($Mg^{2+}$, $Ca^{2+}$, $Ba^{2+}$); le cation d'un métal de transition ($Zn^{2+}$, $Cu^{2+}$); $Al^{3+}$; $Fe^{3+}$; le cation d'une terre rare ($Sc^{3+}$, $Y^{3+}$, $La^{3+}$); un cation organique du type "onium", tels que oxonium, ammonium, pyridinium, guanidinium, amidinium, sulfonium, phosphonium, ces cations organiques étant optionnellement substitués par un ou plusieurs radicaux organiques; un cation organométallique tel que les métallocénium, arène-métallocénium, alkylsilyle, alkylgermanyle ou alkylétain.

De telles membranes comportent cependant plusieurs désavantages importants.

A) Bien que les copolymères formant la membrane soient insolubles dans leur forme ionique, la membrane n'a pas une bonne stabilité dimensionnelle et gonfle de façon significative dans l'eau ou les solvants polaires. Ces copolymères forment des micelles inversées uniquement lorsque chauffés à haute température dans un mélange spécifique eau-alcool, qui, après évaporation, permet de produire un film. Toutefois, ce film régénéré sous forme solide n'a pas de bonnes propriétés mécaniques.

B) Le tétrafluoroéthylène (TFE) est un produit dont la manipulation est très risquée, car sa polymérisation s'effectue sous pression et peut causer des réactions non contrôlée, particulièrement en présence d'oxygène. À cause de la différence de point d'ébullition entre les 2 monomères formant le copolymère et leur différence de polarité, il est difficile d'obtenir un copolymère statistique correspondant au taux d'addition de chaque monomère.

C) Les groupements ioniques en forte concentration sur la chaîne auraient tendance à causer la solubilisation du copolymère. Afin de prévenir ce phénomène, la concentration de groupements ioniques est gardée à un faible taux en ajoutant une importante fraction molaire de monomères de TFE et/ou en augmentant la longueur des chaînes secondaires (n > 1), avec pour résultat que la concentration de groupements d'ions échangeables est de moins de 1 milliéquivalent par gramme. Conséquemment, la conductivité est relativement faible et très sensible au contenu d'eau dans la membrane, particulièrement lorsque cette dernière est acidifiée pour des applications dans une pile à combustible.

D) La pénétration du méthanol et de l'oxygène à travers la membrane est élevée, puisque la portion perfluorocarbonée du polymère permet une diffusion facile des espèces moléculaires, qui vont réagir chimiquement à l'électrode opposée et causer une perte d'efficacité faradaïque, principalement dans les piles à combustible de méthanol.

Les systèmes non fluorés tels que les polyimides sulfonés ou les polyéthers sulfones sulfonés présentent les mêmes inconvénients puisqu'il faut faire un compromis entre la densité de charge, donc la conductivité, et la solubilité ou le gonflement excessif.

## SOMMAIRE DE L'INVENTION

La présente invention concerne un monomère bifonctionnel de formule générale

$$[T\text{-}SO_2\text{-}Y\text{-}SO_2T']^- M^+$$

dans laquelle

- T et $T^+$ sont identiques ou différents et comprennent

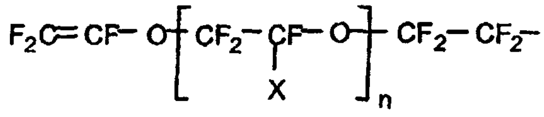

;

,

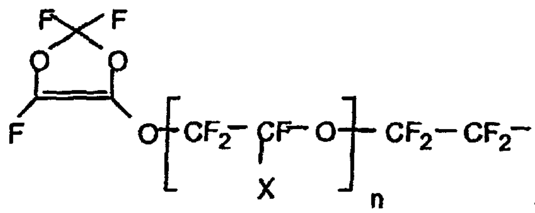

;

$CZ_2$=CZ-;

et

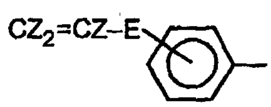

dans lesquels :

- X comprend un halogène ou $CF_3$;
- n varie entre 0 et 10 inclusivement:
- E est absent O, S, $SO_2$; et
- Z est F ou H; et
- $M^+$ comprend un cation inorganique ou organique.
- Y comprend N ou CQ dans lequel Q comprend H, CN, F, $SO_2R^3$, $C_{1-20}$ alkyle substitué ou non-substitué; $C_{1-20}$ aryle substitué ou non-substitué; $C_{1-20}$ alkylène substitué ou non-substitué, dans lesquels le substituant comprend un ou plusieurs halogènes, et dans lesquels la chaîne comprend un ou plusieurs substituants F, $SO_2R$, aza, oxa, thia ou dioxathia; et
- $R^3$ comprend F, $C_{1-20}$ alkyle substitué ou non-substitué; $C_{1-20}$ aryle substitué ou non-substitué; $C_{1-20}$ alkylène substitué ou non-substitué, dans lesquels le substituant comprend un ou plusieurs halogènes.

Dans un mode de réalisation préférentiel, $M^+$ comprend le proton, le cation d'un métal tel un métal alcalin, un métal alcalino-terreux, une terre rare ou un métal de transition; un cation organométallique tel qu'un métallocénium, un arène-métallocénium, un alkylsilyle, un alkylgermanyle ou un alkylétain; ou un cation organique optionnellement substitué par un ou plusieurs radicaux organiques. Des exemples de cations organiques préférentiels incluent un groupement $R''O^+$ (onium), $NR''^+$ (ammonium), $R''C(NHR'')_2^+$ (amidinium), $C(NHR'')_3^+$ (guanidinium), $C_5R''N^+$ (pyridinium), $C_3R''N_2^+$ (imidazolium), $C_2R''N_3^+$ (triazolium), $C_3R''N_2^+$ (imidazolinium), $SR''^+$ (sulfonium), $PR''^+$ (phosphonium), $IR''^+$ (iodonium), $(C_6R'')_3C^+$ (carbonium), dans lesquels R" comprend:

- le proton, les radicaux alkyles, alkényles, oxaalkyles, oxaalkényles, azaalkyles, azaalkényles, thiaalkyles, thiaalkényles, dialkylazo, silaalkyles optionnellement hydrolysables, silaalkényles optionnellement hydrolysables, lesdits radicaux pouvant être linéaires, ramifiés ou cycliques et comprenant de 1 à 18 atomes de carbone;
- les radicaux cycliques ou hétérocycliques aliphatiques de 4 à 26 atomes de carbone comprenant optionnellement au moins une chaîne latérale comprenant un ou plusieurs hétéroatomes tels que l'azote, l'oxygène ou le soufre;
- les aryles, arylalkyles, alkylaryles et alkénylaryles de 5 à 26 atomes de carbone comprenant optionnellement un ou plusieurs hétéroatomes dans le noyau aromatique où dans un substituant.
- les groupes comprenant plusieurs noyaux aromatiques ou hétérocycliques, condensés ou non, contenant optionnellement un ou plusieurs atomes d'azote, d'oxygène, de soufre ou de phosphore;

et lorsqu'un cation organique comporte au moins deux radicaux R" différents de H, ces radicaux peuvent former ensemble un cycle aromatique ou non, englobant optionnellement le centre portant la charge cationique.

L'invention comprend en outre un polymère échangeur d'ions et conducteur solide d'ions obtenu à partir d'un monomère ou un mélange de monomères bifonctionnels tels que définis précédemment. Le monomère ou mélange de monomère peut en outre être copolymérisé avec au moins un monomère monofonctionnel, préférablement de formule $[T\text{-}SO_3]^-M^+$ ou $[T\text{-}SO_2\text{-}Y\text{-}SO_2\text{-}W]^-M^+$ dans lesquels T, Y et $M^+$ sont tels que définis précédemment, Z représente F et W comprend un radical organique monovalent comprenant de 1 à 12 atomes de carbone, un radical organique monovalent comprenant de 1 à 12 atomes de carbone fluoré partiellement ou en totalité, un radical organique monovalent comprenant de 1 à 12 atomes de carbone substitué par un ou plusieurs oxa, aza, thia ou dioxathia et un radical organique monovalent comprenant de 1 à 12 atomes de carbone, fluoré partiellement ou en totalité et substitué par un ou plusieurs oxa, aza, thia, ou dioxathia.

L'invention concerne en outre un procédé de préparation d'un polymère à partir d'un monomère ou mélanges de monomères précités, dans lequel les monomères ou mélanges de monomères sont polymérisés en solution dans un solvant, le polymère formé restant plastifié d'une façon homogène par le solvant. Les monomères ou mélanges de monomères sont préférablement polymérisés sous forme d'émulsion dans des solvants non miscibles.

Le procédé de la présente invention est particulièrement avantageux par rapport aux procédés de l'art antérieur, puisque qu'il permet d'obtenir un polymère qui demeure plastifié d'une manière homogène dans le solvant. Ce phénomène est rare et tout à fait inattendu, et s'explique par les fortes interactions entre les charges et le solvant.

## DESCRIPTION DÉTAILLÉE DE L'INVENTION

La présente invention concerne notamment l'utilisation de perfluoro-di(vinyléthers) portant des fonctions imide ou sulfone hautement dissociés, tels que les di(sulfonylméthane) ou tri(sulfonylméthane), comme base pour l'élaboration de résine échangeuse d'ions réticulée obtenue directement dans une forme finale, par exemple un film ou une fibre creuse (ci-dessous communément appelés "membrane"), et comportant une densité importante de fonctions ioniques, résultant en une conductivité accrue. La polymérisation peut être conduite dans une solution concentrée du monomère sous forme de sel. Les polymères obtenus ne possèdent pas les désavantages des ionomères perfluorés de l'art antérieur, puisqu'ils ont une bonne stabilité dimensionnelle en présence de solvants, incluant l'eau et les solvants polaires, tout en maintenant une excellente conductivité grâce à la concentration élevée de groupements ioniques. De plus, la réticulation forme une excellente barrière face à la diffusion d'espèces moléculaires, en particulier l'oxygène ou le méthanol, de même qu'à d'autres combustibles organiques. La présence de TFE n'est pas nécessaire ou peut être minimisée, réduisant ainsi les risques dans le processus de fabrication. Les polymères peuvent être convertis en membranes extrêmement minces, c'est-à-dire, avec une épaisseur de l'ordre de 50 $\mu$m ou moins, tout en ayant une bonne tenue mécanique, alors que les membranes conventionnelles de même épaisseur n'ont aucune tenue mécanique. Le procédé de la présente invention représente donc une utilisation efficace des monomères en terme de coûts.

Les applications électrochimiques des membranes obtenues à partir des polymères réticulés de la présente invention font appel à des matériaux d'électrodes et/ou des catalyseurs mis en contact intime avec la membrane servant d'électrolyte. Lors de l'utilisation des ces membranes, les matériaux d'électrode peuvent être aisément déposés sur l'un des deux, ou les deux côtés de la membrane lors de sa confection, et ce éventuellement durant l'étape de la polymérisation. Lesdits matériaux d'électrode peuvent également être appliquée sur une membrane déjà formée. Ce revêtement peut s'effectuer par l'application d'une solution d'au moins un monomère selon la présente invention dans un solvant approprié, suivi de la polymérisation, ou encore par l'application d'une solution ou d'une suspension d'un polymère portant éventuellement des fonctions ioniques. Dans tous les cas, les polymères présents aux électrodes présentent avantageusement la fonction de liant des matériaux actifs, conducteurs ou catalyseurs.

Dans un mode de réalisation préférentiel, la fonction imide, i.e., lorsque Y = N, ainsi que les carbanions polysulfonyles, et dans une moindre mesure les anions perfluorosulfonate ayant une forte affinité électronique et étant très dissociés, permettent une augmentation de l'activité catalytique des cations, spécifique pour plusieurs réactions. Les polymères de l'invention sont donc utiles comme support pour des catalyseurs. Sous forme d'un matériau réticulé, la membrane ou le matériau qui la compose par exemple sous forme de poudre ou de granulés, et contenant les cations actifs en catalyse, sont facilement séparés mécaniquement du milieu réactionnel une fois la réaction effectuée. Des exemples de réactions pouvant être catalysées incluent les additions Diels-Alder, les réactions Friedel-Craft, les condensations aldol, les polymérisations cationiques, les estérifications, la formation d'acétals etc.

Les monomères de l'invention peuvent être obtenus par différents procédés. Par exemple, les monomères de type imide s'obtiennent de la façon suivante:

$$2\ TSO_2\text{-}L + [A_2N]^-\ M^+ \Rightarrow 2\ LA + (TSO_2)_2N^-\ M^+$$

ou encore

$$TSO_2\text{-}L + [T'AN]^- M^+ \Rightarrow LA + [(TSO_2)N(SO_2T')]^- M^+$$

dans lesquels L est un groupement labile, par exemple un halogène, un thiocyanate, ou un groupement électrophile contenant au moins un hétéroatome, tels que N-imidazolyle, N-triazolyl, ou $R\text{-}SO_2O\text{-}$ dans lequel R est $C_{1-20}$ alkyle ou $C_{1-20}$ alkylène substitué ou non-substitué, dans lesquels le substituant est un ou plusieurs halogènes; et dans lesquels la chaîne comprend un ou plusieurs substituants choisis parmi aza, oxa, thia, ou dioxathia; et A est l'élément ou la fraction d'élément correspondant au cation $M^+$, et comprend l'hydrogène, un groupement trialkylsilyle, un groupement trialkylétain ou tertioalkyle, dans lesquels le groupement alkyle comprend de 1 à 6 atomes de carbone.

De la même façon, les monomères composés de carbones, i.e., lorsque Y = CQ, sont obtenus à partir de réactions similaires:

$$2\ TSO_2\text{-}L + [A_2CQ)^- M^+ \Rightarrow 2\ LA + [(TSO_2)_2CQ]^- M^+$$

dans lesquels L, T, Q, A et M sont tels que définis ci-dessus.

Le radical tertioalkyle dans la définition de A ci-dessus est avantageux puisqu'il constitue le précurseur d'un alcène s'éliminant du milieu réactionnel et d'un proton. Par exemple, s'il s'agit d'un tertiobutyle, la réaction suivante est observée:

$$(CH_3)_3C\text{-}Y \Rightarrow H\text{-}Y + (CH_3)_2{=}CH_2$$

Le groupement trialkylsilyle est avantageux lorsque le groupement labile L est le fluor, étant donné l'enthalpie élevée de formation du lien Si-F.

Lorsque A est un proton ou un précurseur du proton, tel qu'un radical tertioalkyle, il est avantageux de réaliser la réaction en présence d'une base encombrée, par exemple une base tertiaire. Des exemples de telles bases sont la triéthylamine la di-isopropylamine, la quinuclidine, le 1,4-diazobicyclo[2,2,2]octane (DABCO), la pyridine, les alkylpyridines, les dialkylaminopyridines, les N-alkylimidazoles, imidazo[1,1-a]pyridine, les amidines tels que le 1,5-diazabicyclo[4,3,0]non-5-ène (DBN) ou le 1,8-diazabicyclo[5,4,0]undec-7-ène (DBU), les guanidines tels que la tétraméthylguanidine, la 1,3,4,7,8-hexahydro-1-méthyl-2H-pyrimido-[1,2-a]pyrimidine (HPP).

Dans plusieurs cas, le sel de potassium des monomères de l'invention sont insolubles ou très peu solubles dans l'eau, et peuvent y être précipités à partir de sels plus solubles, c'est-à-dire, les sels $H^+$, $Li^+$ ou $Na^+$, et par la suite purifiés par recristallisation. La recristallisation peut être effectuée dans l'eau, seule ou en mélange avec un solvant miscible, tel que l'acétonitrile, le dioxanne, l'acétone ou le THF.

Les sels d'alkylammonium, particulièrement les sels tétraalkylammonium ou d'imidazolium, sont habituellement insolubles dans l'eau et peuvent donc être extraits à l'aide de solvants variés, préférablement halogénés, tels que le dichlorométhane, le dichloroéthane, le trichloroéthane, le 1,1,1 ,2-tétrafluoroéthane etc.

Il est entendu que toute fonction du monomère pouvant interférer avec la réaction menant à la formation du lien $SO_2\text{-}Y\text{-}SO_2$ est préalablement protégée selon des techniques de protection bien connues de la personne du métier. Par exemple, les groupements perfluorovinyléther peuvent être halogénés, en particulier chlorés ou bromés, pour donner un perhaloéther non réactif. Le perfluorovinyléther est éventuellement régénéré selon différents procédés bien connus de la personne du métier, par exemple, soit par une réaction électrochimique, soit avec un agent de réduction comme la poudre de zinc, un alliage de bronze zinc-cuivre, ou le tétrakis(diméthylamino)éthylène.

Les monomères bifonctionnels suivants illustrent des monomères préférentiels de l'invention.

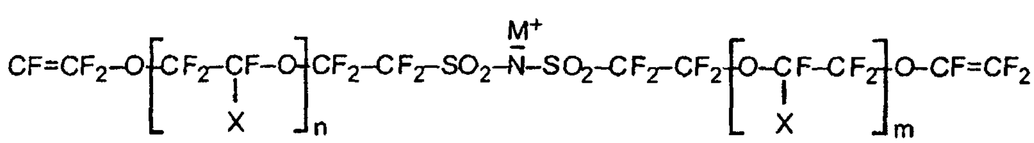

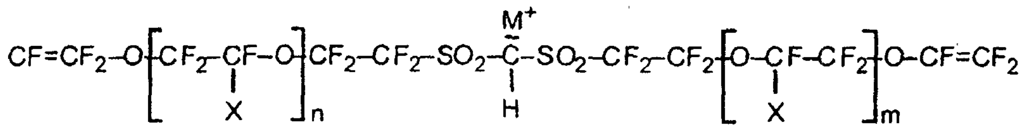

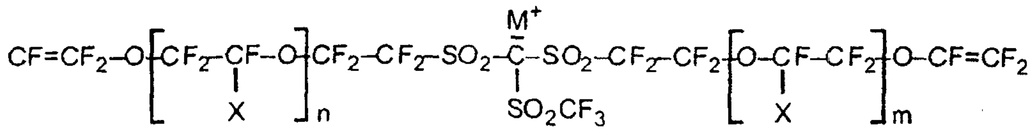

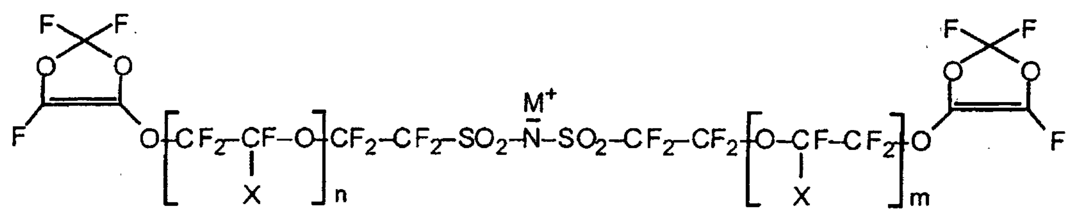

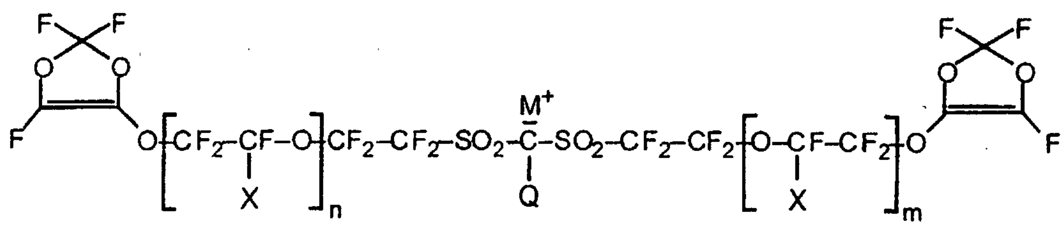

$[CZ_2{=}CZSO_2\text{-}N\text{-}SO_2CZ{=}CZ_2]^- M^+$

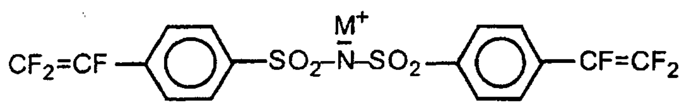

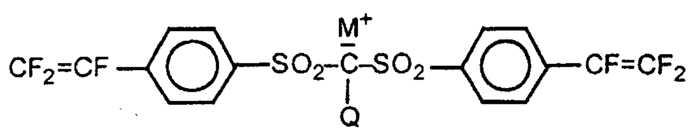

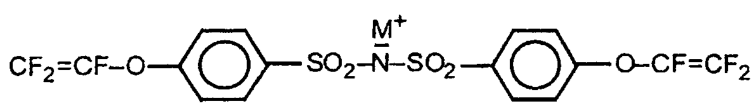

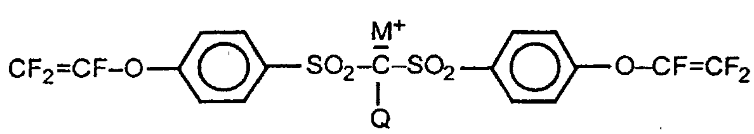

dans lesquelles $M^+$, Z, Q, X et Y sont tels que définis précédemment, et n et m sont identiques ou différents et varient

entre 0 et 10 inclusivement.

En pratique, les membranes échangeuses d'ions sont obtenues par homo- ou copolymérisation des monomères bifonctionnels de la présente invention. Pour les copolymères, les comonomères sont avantageusement choisis parmi les sels de monomères monofonctionnels de formule générale:

$$[T'\text{-}SO_3]^- M^-$$

ou

$$[T'\text{-}SO_2\text{-}Y\text{-}SO_2\text{-}W]^- M^-$$

dans lesquels T', Y, $M^+$ et W sont tels que définis précédemment.

Des exemples de monomères monofonctionnels préférentiels de l'invention pour une copolymérisation incluent:

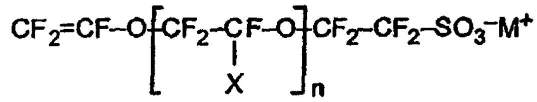

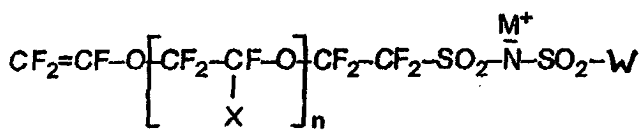

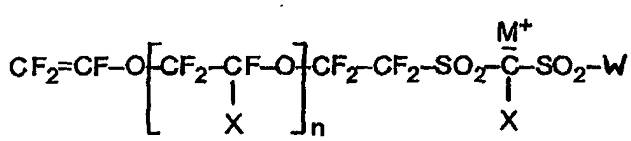

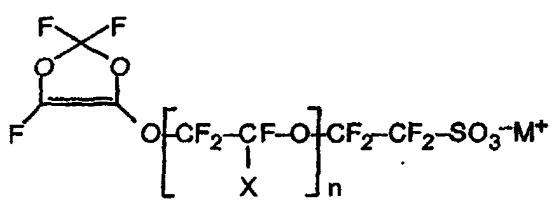

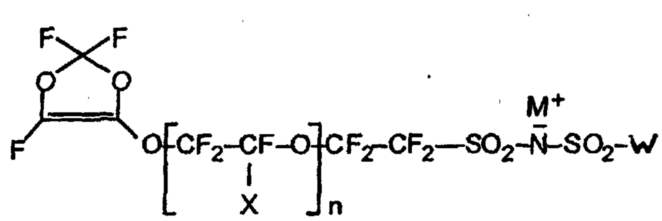

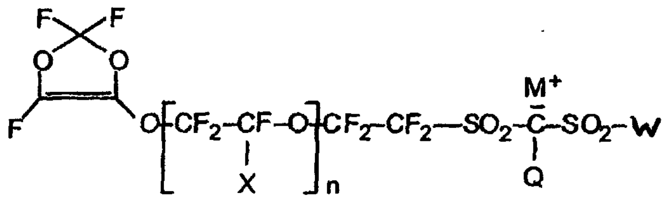

$CF_2=CFSO_3^-M^+$

$CF_2=CFSO_3^-M^+$

$[CF_2=CFSO_2\text{-}N\text{-}SO_2W]^-M^+$

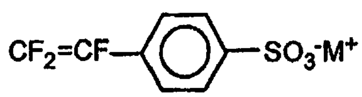

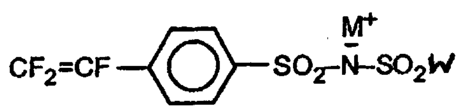

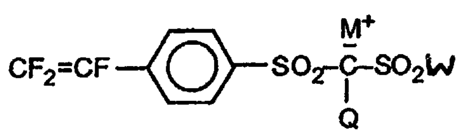

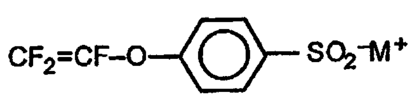

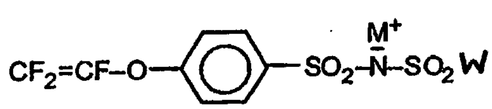

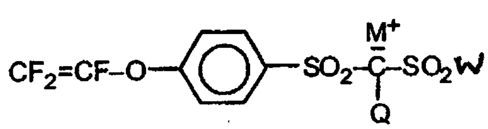

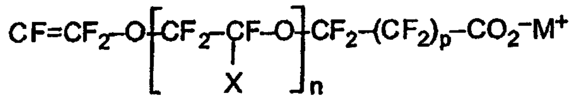

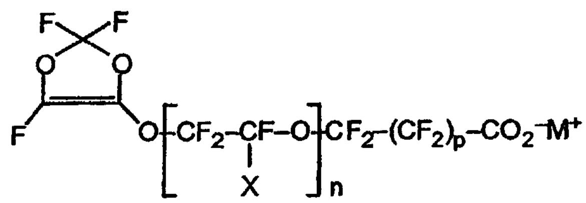

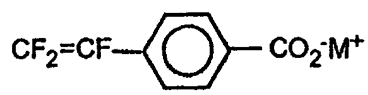

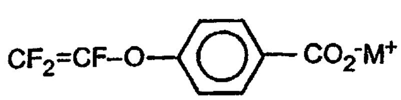

dans lesquelles M, O, X, W et n sont tels que défini précédemment.

De façon avantageuse, les réactions de polymérisation et copolymérisation se font dans un solvant des monomères. Ces derniers sont généralement solubles dans la plupart des solvants polaires usuels, i.e., eau, alcools aliphatiques inférieurs, acétone, méthyléthylcétone, cétones cycliques, carbonates d'éthyle et propyle, Y-butyrolactone, N-alkylimidazole, fluoroalcanes, et leurs mélanges. En outre, le choix de M permet d'optimiser la solubilité des monomères, grâce aux interactions cation-solvant Il est entendu qu'après que la polymérisation a été complétée, les échanges de M se font selon des techniques conventionnelles utilisées dans le domaine des résines échangeuses d'ions. Des additifs solides organiques ou inorganiques, sous forme de poudre ou de fibres, peuvent être ajoutés à l'étape de fabrication pour améliorer les propriétés mécaniques des polymères, agir comme agent de formation de pores, ou comme support de catalyseur (e.g., platine déposé sur des particules de carbone).

Les exemples suivants sont fournis afin d'illustrer des modes de réalisations préférentiels de l'invention.

**Exemples**

Exemple 1

Sous atmosphère d'azote, 25 g de chlorure de p-iodobenzène sulfonyle sont traités par 1.44 g de nitrure de lithium dans 125 ml de DMF anhydre. Après 24 heures de réaction sous agitation mécanique constante, le mélange réactionnel est filtré et le solvant est évaporé sous pression réduite à 80°C. Le résidu solide contenant le sel de lithium de la 4-iodophénylsulfonimide est dissous dans 100 ml d'eau, filtré et acidifié par de l'acide sulfurique jusqu'à obtention d'un pH de 1. La 4-iodophénylsulfonimide est extraite par quatre aliquotes de 100 ml d'éther, les phases organiques sont subséquemment combinées et l'éther est évaporé. La 4-iodophénylsulfonimide est purifiée par cristallisation dans l'eau et le sel de zinc est préparé par action du carbonate de zinc en quantité stoechiométrique. Le sel est séché sous vide à 80°C.

L'organozincique $CF_2$=CFZnBr est préparé sous argon dans le DMF selon la procédure publiée dans J. Organic Chemistry, **53**, 2714, (1988) à partir de $CF_2$=CFBr (10 g) dans un réacteur thermostaté. 18 g du sel de zinc tel que préparé précédemment dans la DMF sont ajoutés à la solution de l'organométallique mélangé à 160 mg de benzylidèneacétone palladium (0) et 190 mg de triphénylphosphine agissant comme co-catalyseur, en maintenant la température en dessous de 65 °C. La réaction est poursuivie pendant 4 heures à cette température et le solvant est évaporé sous pression réduite à 80°C. Le résidu solide est repris par l'eau, filtré et traité par 10 g de carbonate de potassium dans 100 ml d'eau. La suspension blanche contenant le carbonate de zinc est évaporée sous pression réduite à 60°C. Le sel de potassium est extrait par le mélange acétonitrile-diméthoxyéthane (50:50 v/v), et le solvant est évaporé. Le sel de potassium :

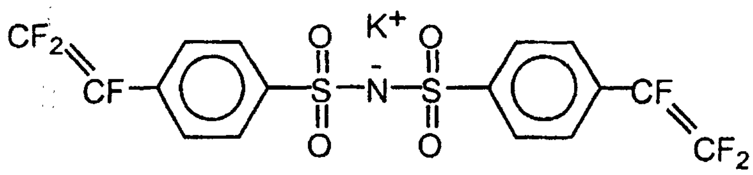

est purifié par recristallisation dans l'eau et transformé en sel de lithium par double échange avec le tétrafluoroborate de lithium dans l'acétonitrile dans lequel $KBF_4$ est insoluble.

Exemple 2

Une solution de 1 g du sel de lithium de l'exemple 1, 10 g de 4-trifluorovinylbenzène sulfonate de lithium, 250 mg d'Irgacure 651® dans 35 ml d'un mélange de carbonate de propylène et de diglyme (bis[méthoxyéthyléther]) (50: 50 v/v) sont épandus à l'aide d'un gabarit sous forme de film de 180 microns d'épaisseur sur un support de polypropylène. Sous balayage d'argon, la solution soumise au rayonnement UV produit par une lampe de type Hanovia® ayant son maximum d'émission à 254 nanomètres de manière à ce que l'éclairement corresponde à de 80 $mWcm^{-2}$. La solution se polymérise alors sous forme d'un gel élastique. La polymérisation est poursuivie pendant 5 minutes puis le film est décollé de son support et lavé à l'eau puis à l'acide nitrique 2M à 60°C afin d'éliminer les solvants organiques et les résidus de polymérisation. La conductivité de la membrane mesurée entre 60 et 100 % d'humidité est supérieure à $10^{-2}$ $Scm^{-1}$. Les dimensions de la membrane sont stables dans un grand domaine de taux d'humidité relative du fait de la forte concentration de noeuds de réticulation.

Exemple 3

30 g de chlorure de 4-iodobenzènesulfonyle, 15 g de trifluorométhanesulfonamide et 23 g de 1,4-diazabicyclo [2,2,2]octane sont dissous dans 200 ml d'acétonitrile anhydre et maintenus sous agitation magnétique pendant 48 heures à 25°C. La suspension est filtrée et l'acétonitrile est évaporé. Le résidu solide est repris par le minimum d'eau auquel est ajouté 100 ml d'une solution saturé de KCl. Le précipité du sel de potassium est séparé par filtration et purifié par recristallisation dans l'eau bouillante. D'une manière similaire à celle de l'exemple 2, l'iode en position para est remplacé par le groupement $CF_2$=CF par couplage de l'organozincique $CF_2$=CFZnBr en présence de catalyseur à base de palladium. Le sel de potassium

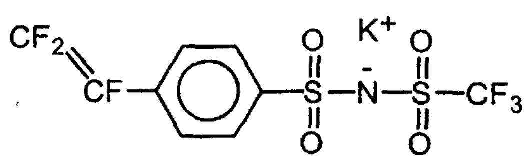

est purifié par recristallisation dans l'eau et transformé en sel de lithium. Une solution de 8.8 g de ce sel et 1 g du monomère de l'exemple 1 sous forme de sel de lithium sont dissous dans 40 ml de γ-butyrolactone, épandus sous forme de film de 150 microns d'épaisseur sur un support de polypropylène et polymérisés dans les conditions de l'exemple 2. Le film est décollé de son support et lavé à l'eau et à l'acide nitrique 2M à 60°C de manière à éliminer la γ-butyrolactone et effectuer l'échange $Li^+$ => $H^+$. D'une manière similaire, le mélange de sels en proportions identiques dans la γ-butyrolactone en suspension dans le décane sont polymérisés par le persulfate de tridécylméthyl ammonium agissant comme tensioactif et générateur de radicaux libres à 60°C. Un latex de particules de dimension proche de 20 μm est ainsi obtenu, séparé par filtration et traité comme précédemment pour effectuer l'échange $Li^+ \Rightarrow H^+$.

Exemple 4

50 g de fluorure de perfluorovinyloxy-éthanesulfonyle $CF_2=CFOCF_2CF_2SO_2F$ dans 300 ml le tétrachlorure de carbone sont traités par un excès de chlore dans un récipient de quartz sous éclairage UV. Le produit d'addition sur la double liaison $CF_2(Cl)CF(Cl)OCF_2CF_2SO_2F$ est purifié par distillation. Dans un récipient en polyethylène haute densité à parois épaisses (Nalgène®) sont ajoutés 35 g de $CF_2(Cl)CF(Cl)OCF_2CF_2SO_2F$ tel que préparé précédemment, 125 ml de THF anhydre et 1.74 g de nitrure de lithium et 36 cylindres de broyage en zircone (@ 1 $cm^3$). Après 24 heures d'agitation dans un broyeur à rouleaux, 10 g de poudre d'alliage zinc-cuivre (10% Cu) sont ajoutés et l'agitation est poursuivie pendant 24 heures supplémentaires. Le produit final est filtré et le solvant est évaporé sous pression réduite à 40°C. Le résidu est repris par une solution à 10% de chlorure de potassium dans l'eau. Le précipité est lavé, filtré et recristallisé dans un mélange eau-éthanol (50:50 v/v). Le sel de lithium $Li[(CF_2=CFOCF_2CF_2SO_2)_2N]$ est obtenu par échange par $LiBF_4$ dans le triglyme.

Le perfluorovinyloxy-éthylsulfonate de lithium $CF_2=FOCF_2CF_2SO_3Li$ est préparé par une procédure similaire à partir fluorure du perfluorovinyloxy-éthanesulfonyle par traitement par le triméthylsilanoate de lithium dans le triglyme et filtré.

Exemple 5

Une membrane perfluorée réticulée est préparée en phase homogène par copolymérisation de 4 g du sel de lithium $Li[(CF_2=CFOCF_2CF_2SO_2)_2N]$ de l'exemple 4, avec 24 g de perfluorovinyloxy-éthanesulfonate de lithium dans 60 ml de triglyme. 1.5 g de nanoparticules de silice filmée (taille moyenne 70 Å) sont ajoutées et dispersées sous agitation mécanique dans un mélangeur à boulets. L'amorceur radicalaire est le peroxyde de trichlooacétyle en proportion de 1% molaire par rapport aux monomères. La solution est épandue sur un support de polypropylène de manière à former un film de 100 microns d'épaisseur. L'étape de polymérisation/réticulation est obtenue par chauffage à 80°C sous atmosphère d'azote désoxygéné. Le film obtenu est décollé de son support et lavé à l'eau et à l'acide nitrique 2M à 60°C de manière à éliminer les solvants organiques et les résidus de polymérisation ainsi qu'effectuer le remplacement des cations $Li^+$ par $H^+$. La conductivité de la membrane à 95% d'humidité est d'environ $10^{-2}$ $Scm^{-1}$. La perméation du méthanol est inférieure d'un ordre de grandeur par rapport à une membrane de Nafion 117® d'épaisseur similaire.

Exemple 6

17.5 g de fluorure de perfluorovinyloxy-éthanesulfonyle $CF_2(Cl)CF(Cl)OCF_2CF_2SO_2F$ traités par le chlore sous rayonnement UV sont versés dans un récipient en Nalgène® et sont ajoutés 100 ml de THF anhydre et 1.2 g de carbure d'aluminium $C_3Al_4$ ainsi que 36 cylindres de broyage en zircone (environ 1 $cm^3$). Après 48 heures d'agitation dans un broyeur à rouleaux, sont ajoutés 10 g de poudre de zinc et l'agitation est poursuivie pour 48 heures supplémentaires. Le produit final est filtré et le solvant est évaporé sous pression réduite à 40°C. Le résidu est repris par une solution à 10% de chlorure de potassium dans l'eau. Le précipité est lavé, filtré et recristallisé dans un mélange eau-éthanol (50:50 v/v). Par échange avec $LiBF_4$ le sel de lithium $Li[(CF_2=CFOCF_2CF_2SO_2)_2CH]$ est obtenu. Ce monomère difonctionnel peut se polymériser d'une manière similaire à celui de l'exemple 4, et est avantageusement utilisé lorsque des fonctions fortement acide mais peu hygroscopiques sont préférées.

Exemple 7

Le fluorure de perfluoro(4-méthyl-3,6-dioxaoct-7-ène)sulfonyle (Synquest Research Chemicals Fl, USA) est traité par le nitrure de lithium dans les conditions de l'exemple 4 après protection de la double liaison par le brome. Le sel de lithium $Li[(CF_2=CFOCF_2CF(CF_3)OCF_2CF_2SO_2)_2N]$ est obtenu après réduction par le zinc et purification au stade du sel de potassium obtenu comme intermédiaire. Le perfluoro(4-méthyl-3,6-dioxaoct-7-ène)sulfonate de lithium est obtenu d'une manière similaire par réaction du silanoate de lithium sur le fluorure de sulfonyle dans le dibutyldiglyme ($[C_4H_9OC_2H_4]_2O$). Une membrane réticulée est obtenue par polymérisation d'un mélange de 3 g du monomère divinylique et de 18 g du perfluorosulfonate monovinylique, 800 mg de silice fumée (7 nm) dans 50 ml de dibutyldiglyme. La solution est épandue sous forme de film et la polymérisation est effectuée à l'aide du photoamorceur Irgacure 651®. Sous balayage d'argon, la solution soumise au rayonnement UV dans les conditions de l'exemple 2. La membrane est lavée à l'éthanol puis à l'eau et les ions lithium sont échangés par HCl 5M dans l'eau.

Exemple 8

Le fluorure de perfluoro(4-méthyl-3,6-dioxaoct-7-ène)sulfonyle de l'exemple 7 est traité par le carbure d'aluminium dans les conditions de l'exemple 6. Après traitement par le carbonate de potassium et élimination de l'hydroxyde d'aluminium formé, le sel de lithium $Li[(CF_2{=}CFOCF_2CF(CF_3)OCF_2CF_2SO_2)_2CH]$ est obtenu après purification du sel de potassium et échange en présence de $LiBF_4$ dans le THF. Des résultats similaires sont obtenus en utilisant à la place du carbure d'aluminium le réactif de Nysted ($Zn_3Br_2(CH_2)_2$, THF). Comme son analogue azoté, ce monomère est susceptible d'homopolymèriser ou de copolymériser avec le tétrafluoroéthylène ou les monomères monofonctionnels ioniques. Un copolymère réticulé avec le perfluoro(4-méthyl-3,6-dioxaoct-7-ène) sulfonate de lithium (10 : 90 molaire) est obtenu d'une manière similaire à celle de l'exemple 7.

Exemple 9

À 3.73 g de trifluorométhylméthylsulfone $CF_3SO_2CH_3$ dans 50 ml de THF sont ajoutés à 0°C 600 mg d'hydrure de sodium puis 3.15 ml de chlorotriméthylsilane. Après précipitation du chlorure de sodium formé, le liquide surnageant est filtré et sont ajoutés 20 ml d'un solution 2.5 M de butyllithium dans l'hexane. Le produit d'addition du chlore sur la double liaison vinylique $CF_2(Cl)CF(Cl)OCF_2CF_2SO_2F$ est préparé comme pour l'exemple 4. Dans un récipient en polyéthylène haute densité à parois épaisses (Nalgène®) sont ajoutés le dérivé dilithié de la sulfone dans le THF tel que préparé, 6.7 ml de tétraméthyléthylène diamine (TMDA) et 17.5 g de $CF_2(C1)CF(C1)OCF_2CF_2SO_2F$ obtenu par addition de chlore sur la double liaison du perfluorovinyloxy-éthanesulfonyle d'une manière similaire à celle de l'exemple 4. 36 cylindres de broyage en zircone (environ 1 $cm^3$) sont inclus pour favoriser la réaction hétérogène solide-liquide. Après 24 heures d'agitation dans un broyeur à rouleaux, 5 g de poudre d'alliage zinc-cuivre (10% Cu) sont ajoutés et l'agitation est continuée pendant 24 heures supplémentaires. Le produit final est filtré et le solvant est évaporé sous pression réduite à 40°C. Le résidu est repris par une solution à 10% de chlorure de potassium dans l'eau. Le précipité est lavé, filtré et recristallisé dans un mélange eau-éthanol (50:50 v/v). Le sel de lithium $Li[(CF_2CFOCF_2CF_2SO_2)_2CSO_2CF_3$ est obtenu par échange par $LiBF_4$ dans le triglyme. Les monomères monofonctionnels bis(trifluométhanesulfonyl-(trifluoperfluorovinyloxy-éthylsulfonyl)méthylure de lithium Li $[CF_2{=}CFOCF_2CF_2SO_2C(SO_2CF_3)_2$ et trifluométhanesulfonyl-(trifluoperfluorovinyloxy-éthylsulfonylimidure de lithium $Li[CF_2{=}CFOCF_2CF_2SO_2N(SO_2CF_3)$ peuvent être obtenu d'une manière similaire par action de $CF_2(Cl)CF(Cl)OCF_2CF_2SO_2F$ préparé à l'exemple 4 sur les dérivés dilithiés de la disufone $Li_2C(SO_2CF_3)_2$ et de la sulfonamide $Li_2NSO_2CF_3$ respectivement.

Exemple 10

À partir de du fluorure de perfluoro(4-méthyl-3,6-dioxaoct-7-ène)sulfonyle de l'exemple 8 sont obtenus par action des réactifs de l'exemple 9 :

$Li[(CF_2{=}CFOCF_2CF(CF_3)OCF_2CF_2SO_2)_2CSO_2CF_3]$ difonctionnel;
$Li[CF_2{=}CFOCF_2CF(CF_3)OCF_2CF_2SO_2C(SO_2CF_3)_2]$ monofonctionnel;
$Li[CF_2{=}CFOCF_2CF(CF_3)OCF_2CF_2SO_2N(CSO_2CF_3)]$ monofonctionnel.

Ces monomères permettent de préparer des copolymères réticulés incorporant au moins le monomère difonctionnel et un des monomères monofonctionnels choisi parmi ceux des exemples 7, 9 ou 10 ayant en commun des fonctions perfluorovinyléther de réactivité identique.

Exemple 11

Le sel de l'exemple 7 $Li[(CF_2{=}CFOCF_2CF(CF_3)OCF_2CF_2SO_2)_2N]$ est traité par le chlorure de 1-méthyl-3-éthyl-imidazolium en solution dans l'eau. Un liquide visqueux se décante et est extrait au dichlorométhane pour donner

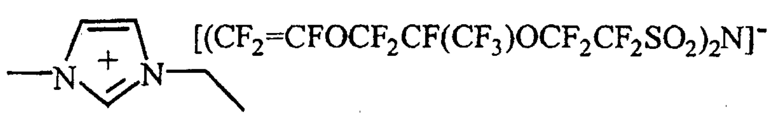
$[(CF_2{=}CFOCF_2CF(CF_3)OCF_2CF_2SO_2)_2N]^-$

Selon la même méthode, le sulfonate monofonctionnel :

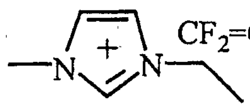
$CF_2{=}CFOCF_2CF(CF_3)OCF_2CF_2SO_3^-$

est préparé par échange dans l'eau, ainsi que sel de l'amorceur radicalaire azobis(2-imidazolidium-2-méthyl-propane):

$2\ CF_2{=}CFOCF_2CF(CF_3)OCF_2CF_2SO_3^-$

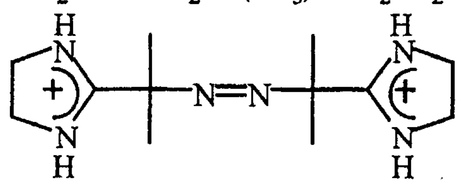

Les sels sont mélangés dans les rapports molaires 8:91:1 monomère difonctionnel : monomère monofonctionnel : amorceur radicalaire. Le mélange visqueux est épandu sur une feuille de polypropylène maintenue à 40°C de manière à former une couche de 35 µm d'épaisseur et le tout est porté à 80°C pendant 2 heures sous balayage d'azote. La membrane obtenue est décollée de son support et la polymérisation est complétée par deux heures de traitement à 100°C. Les ions imidazolium sont échangés par les ions sodium par traitement par une solution de soude 1 M dans l'éthanol au reflux pendant 4 heures, amenant la dégradation du cation organique. Les ions sodium sont à leur tour échangés par des protons par immersion dans un extracteur de type Soxhlet par solution aqueuse d'acide chlorhydrique à la composition azéotropique (20.3% en poids). Le même monomère difonctionnel est copolymérisé avec le monomère monofonctionnel de l'exemple 10, $Li[CF_2{=}CFOCF_2CF(CF_3)OCF_2CF_2SO_2N(CSO_2CF_3)]$ et l'amorceur radicalaire précédemment utilisé. La copolymérisation est effectuée en émulsion inverse dans la décaline sous agitation mécanique forte, les rapports des composants actifs étant maintenant 25:74:1. Le système est purgé par de l'argon et après deux heures à 90°C, les billes de polymère sont séparées par filtration, lavées et échangées comme précédemment.

Exemple 12

17.5 g d'acide 4-hydroxybenzène sulfonique commercial sont traités par 4 ml de soude 4M dans l'eau. Le sel est séché sur un évaporateur rotatif puis par déshydratation azéotropique. 19.5 g du sel obtenu $Na_2(O\Phi SO_3)$ ($\Phi$ = para -$C_6H_4$-) sont mis en suspension dans 100 ml de DMF avec 10.75 ml de 1,2-dibromo tétrafluoroéthane et le mélange est porté à 80°C dans un réacteur Parr® purgé sous azote au préalable sous argon et la réaction est poursuivie pendant 4 heures. Le sel $Na(BrCF_2CF_2O\Phi SO_3)$ est séparé par filtration et le chlorure de sulfonyle est préparé par action du chlorure de thionyle dans l'acétonitrile catalysé par le DMF. L'imidure $Na(BrCF_2CF_2O\Phi SO_2)N$ est préparée à 0°C par addition de soude sur une suspension du chlorure d'acide dans un solution aqueuse de chlorure d'ammonium selon l'équation

$$2\ BrCF_2CF_2O\Phi SO_2C1 + NH_4Cl + 4\ NaOH \Rightarrow 3\ NaCl + 2\ H_2O + Na(BrCF_2CF_2O\Phi SO_2)_2N$$

Dans un récipient en polyéthylène haute densité à parois épaisses (Nalgène®) sont ajoutés 14 g (709.15023) g de l'imidure $Na(BrCF_2CF_2O\Phi SO_2)_2N$ tel que préparé précédemment, 100 ml de DMF anhydre et 4 g de poudre d'alliage zinc et 25 cylindres de broyage en zircone (environ 1 $cm_3$). Après 24 heures d'agitation dans un broyeur à rouleaux, le solvant est évaporé sous pression réduite à 80°C. Le résidu est repris par une solution saturée de chlorure de potassium dans l'eau et le précipité de $K(CF_2CF_2O\Phi SO_2)_2N$ est purifié par cristallisation dans l'eau. Ce sel peut se polymériser par amorçage radicalaire ou thermique ou être inclus dans un copolymère avec le monomère $Na(CF_2CF_2O\Phi SO_3)$ obtenu par réduction par le zinc de $Na(BrCF_2CF_2O\Phi SO_3)$.

Exemple 13

Une pile à combustible expérimentale est fabriquée à partir d'une membrane préparée à l'exemple 3. Une dispersion nanométrique de platine sur support de carbone (Degussa, Allemagne) est appliquée de part et d'autre de

la membrane par une technique de sérigraphie à partir d'une dispersion du carbone platiné dans une solution colloïdale (5% w/w) de Nafion 117® dans un mélange d'alcools légers (Aldrich). Le système est traité à 130°C en appliquant une pression de 20 $Kgcm^{-2}$ pour assurer la cohésion des particules de Nafion®. Un collecteur de papier de carbone (feutre de fibres de carbone non tissées) est interposé entre les électrodes et les collecteurs de courants en acier inoxydable rainuré pour assurer la distribution des gaz. La pile expérimentale est testée avec une alimentation d'hydrogène saturé en vapeur d'eau à 80°C et d'oxygène les deux gaz étant à pression ordinaire. La tension en circuit ouvert est de 1.2 V et la courbe courant tension mesurée sur ce montage indique 1 $Acm^{-2}$ sont obtenus à la tension de 0.65 V.

Exemple 14

Une pile à combustible expérimentale est fabriquée à partir d'une membrane préparée à l'exemple 5. L'électrode de carbone platiné est appliquée de part et d'autre de la membrane par sérigraphie d'une suspension (30% en poids) de ce matériau dans une solution dans le diglyme comprenant A) 15% en poids du monomère de l'exemple 4 $Li[(CF_2=CFOCF_2CF_2SO_2)_2N]$; B) 15% du monomère monofonctionnel de l'exemple 10 $Li[CF_2=CFOCF_2CF(CF_3)OCF_2CF_2SO_2N(CSO_2CF_3)]$; et C) 1% en poids de l'amorceur de l'exemple 11.

La polymérisation du monomère de l'électrodes est obtenue par chauffage sous atmosphère d'azote à 90 °C pendant deux heures. L'assemblage électrodes / membrane est rincée abondamment dans une solution aqueuse d'acide chlorhydrique 2M pour éliminer les solvants organiques, le monomère non réagi et les oligomères, ainsi qu'assurer l'échange des ions $Li^+$ du polymère de l'électrode par des protons. La pile à combustible expérimentale utilisant cet assemblage a une tension en circuit ouvert de 1.2 V et la courbe courant tension mesurée sur ce montage indique 1 $Acm^{-2}$ sont obtenus à la tension de 0.72 V. Le remplacement du platine de l'électrode négative par un alliage platine-ruthénium 50:50 permet l'utilisation comme combustible de méthanol avec une densité de courant de 150 $mAcm^{-2}$ à une tension de 0.6 V à 100 °C. La perméation du méthanol dans ces conditions est inférieure à 5 $\mu moles.cm^{-2}s^{-1}$.

Exemple 15

L'assemblage électrodes/électrolyte d'une pile à combustible expérimentale est réalisé en une seule étape par co-extrusion des trois couches correspondante sous forme de monomères subissant une co-polymérisation/réticulation. La partie centrale est une dispersion de nanoparticules de silice (1.5 g) dans une solution de $Li[(CF_2=CFOCF_2CF_2SO_2)_2N]$ (4 g), de $Li[(CF_2=CFOCF_2CF_2SO_3]$ (24 g) dans 40 ml de triglyme et 1 g de l'amorceur radicalaire de l'exemple 11. Les électrodes sont constituées d'une dispersion de carbone platiné (10 g), de carbonate de calcium en grains micrométriques (10 g), de $Li[(CF_2=CFOCF_2CF_2SO_2)_2N]$, (2 g), de $Li[(CF_2=CFOCF_2CF_2SO_3]$ (12 g) dans 40 ml de triglyme et 0.8 g de l'amorceur radicalaire de l'exemple 11. Les épaisseurs d'extrusions sont ajustées à 60 μm pour l'électrolyte et 30 um pour chacune des électrodes. La polymérisation est immédiatement effectuée après extrusion par chauffage à 80°C pendant 4 heures sous azote. L'assemblage est traité dans un appareil de type Soxhlet par de l'acide chlorhydrique à la composition azéotropique pour échanger les ions métalliques. La dissolution de carbonate de calcium crée une porosité favorables aux échanges gazeux de l'électrodes. L'assemblage co-extrudé ainsi constitué peut-être découpé au dimensions voulues pour constituer des éléments de pile à combustibles modulaire par ajout des collecteurs de courant et des amenées de gaz.

Exemple 16

L'électrolyse du chlorure de sodium est effectuée dans une cellule à deux compartiments séparés par une membrane telle que préparée à l'exemple 7, l'anode étant du type DSA (Dimensionnally Stable Electrode) et constituée de titane recouvert d'une couche d'oxyde de ruthénium $RuO_2$ en contact avec la membrane, la cathode étant en nickel. La chute ohmique pour 2 $Acm^{-2}$ est de 0.4V et la perméation des ions OH- à travers la membrane est inférieure à 9 $\mu moles.cm^{-2}s^{-1}$.

Exemple 17

La membrane de l'exemple 4 est utilisé pour le préparation d'ozone par électrolyse de l'eau sur une anode de dioxyde de plomb, la cathode est une grille de platine, les deux électrodes étant plaquées sur la membrane dont le côté cathodique est immergé dans l'eau. Le rendement faradique en ozone et de 24% sous 5V.

Exemple 18

Les résines échangeuses d'ions poreuses préparées aux exemples 3 et 11 sont utilisées comme catalyseurs de réactions chimiques. Sous forme protonique active après déshydratation sous vide, la résine catalyse les réactions

de Friedel et Craft, les estérifications, les acétalisations etc. À un mélange équimoléculaire d'anisole et d'anhydride acétique sont ajoutés 3% en poids de la résine de l'exemple 3 sous forme acide. La réaction de formation de la 4-méthoxyacétophénone est complète en 45 minutes à température ordinaire.

L'échange des protons pour les ions de transition et les métaux des terres rares, en particulier $La^{+3}$ et $Y^{+3}$ donne un catalyseur pour les réactions de Friedel et Craft et de cross-aldolisation. À un mélange équimoléculaire de cyclopentadiène et de vinyl-méthyl cétone (10 mmoles dans 30 ce de dichlorométhane sont ajoutés 5% en poids de la résine de l'exemple 11 sous forme $Y^{3+}$ et séchée sous vide à 60°C. La réaction de formation du composé de condensation de Diels-Alder est complète à 25°C en 30 minutes, le rapport endo/exo étant proche de 90:10.

Dans les deux cas, le catalyseur est éliminé par simple filtration et réutilisable.

## Revendications

**1.** Monomère bifonctionnel de formule générale $[T\text{-}SO_2\text{-}Y\text{-}SO_2T']^-M^+$ dans laquelle :

- -Y comprend N ou CQ dans lequel Q comprend H, CN, F, $SO_2R^3$, $C_{1-20}$ alkyle substitué ou non-substitué; $C_{1-20}$ aryle substitué ou non-substitué; $C_{1-20}$ alkylène substitué ou non-substitué, dans lesquels le substituant comprend un ou plusieurs halogènes, et dans lesquels la chaîne comprend un ou plusieurs substituants F, $SO_2R$, aza, oxa, thia ou dioxathia, avec $R_3$ qui comprend F, $C_{1-20}$ alkyle substitué ou non-substitué; $C_{1-20}$ aryle substitué ou non-substitué; $C_{1-20}$ alkylène substitué ou non-substitué, dans lesquels le substituant comprend un ou plusieurs halogènes;
- T et T' sont identiques ou différents et comprennent :

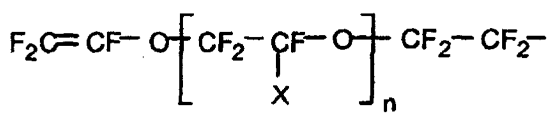
;

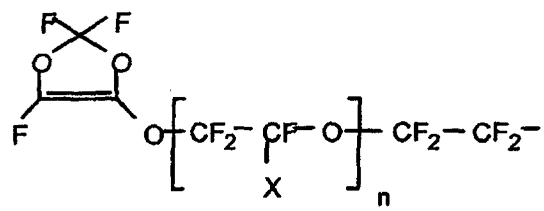
;

$CZ_2$=CZ-; et

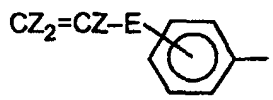

dans lesquels :

- X comprend un halogène ou $CF_3$;
- n varie entre 0 et 10 inclusivement;
- E est absent, O, S, $SO_2$; et
- Z est F ou H ; et
- M+ comprend un cation inorganique ou organique.

**2.** Monomère selon la revendication 1 de formule :

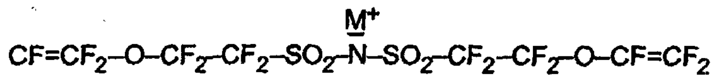 ;

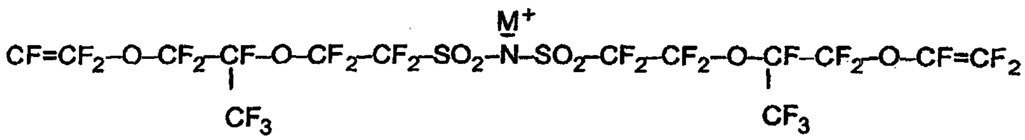 ;

$[CF_2=CFSO_2\text{-}N\text{-}CFSO_2\text{-}CF_2]M^+$;

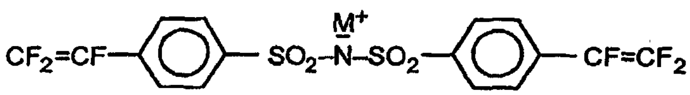

;et
ainsi que leurs mélanges.

3. Polymère échangeur d'ions et conducteur solide d'ions obtenu à partir d'un monomère ou un mélange de monomères selon la revendication 1 ou 2.

4. Polymère selon la revendication 3 dans lequel le monomère bifonctionnel est copolymérisé avec au moins un monomère monofonctionnel de formule $[T\text{-}SO_3]M^+$ ou $[T\text{-}SO_2\text{-}Y\text{-}SO_2\text{-}W]M^+$ dans lesquels T, Y et $M^+$ sont tels que définis dans la revendication 1, Z représente F et W comprend un radical organique monovalent comprenant de 1 à 12 atomes de carbone, un radical organique monovalent comprenant de 1 à 12 atomes de carbone fluoré partiellement ou en totalité, un radical organique monovalent comprenant de 1 à 12 atomes de carbone substitué par un ou plusieurs oxa, aza, thia ou dioxathia et un radical organique monovalent comprenant de 1 à 12 atomes de carbone, fluoré partiellement ou en totalité et substitué par un ou plusieurs oxa, aza, thia, ou dioxathia.

5. Polymère selon la revendication 4 dans lequel au moins un monomère monofonctionnel est de formule :

$$CF_2=CF\text{-}SO_3^-M^+;$$

ou

$$CF_2=CF\text{-}O\text{-}CF_2CF_2SO_3^-M^+;$$

$$CF_2=CF\text{-}O\text{-}CF_2CF(CF_3)O\text{-}CF_2CF_2SO_3^-M^+;$$

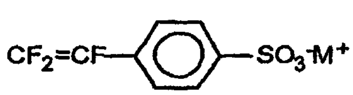

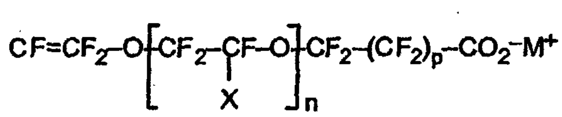

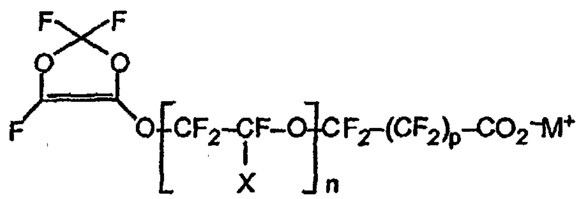

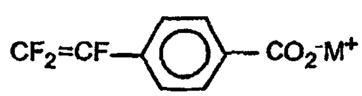

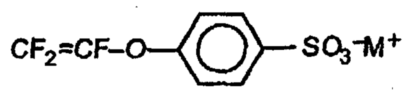

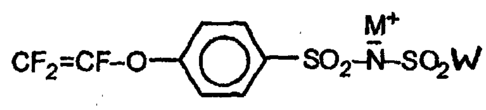

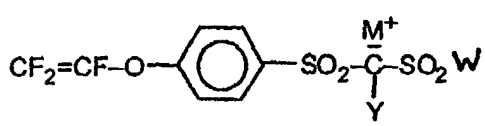

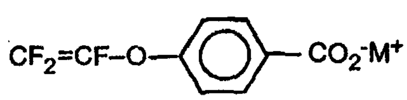

et leurs mélanges, et dans lesquels M, X et n sont tels que définis dans la revendication 1, et W comprend un radical organique monovalent comprenant de 1 à 12 atomes de carbone, un radical organique monovalent comprenant de 1 à 12 atomes de carbone fluoré partiellement ou en totalité, un radical organique monovalent comprenant de 1 à 12 atomes de carbone substitué par un ou plusieurs oxa, aza, thia ou dioxathia et un radical organique monovalent comprenant de 1 à 12 atomes de carbone, fluoré partiellement ou en totalité et substitué par un ou plusieurs oxa, aza, thia, ou dioxathia.

**6.** Procédé de préparation d'un polymère à partir de monomères ou mélanges de monomères selon la revendication 1, dans lequel les monomères ou mélanges de monomères sont polymérisés en solution dans un solvant, le polymère formé restant plastifié d'une façon homogène par le solvant.

7. Procédé selon la revendication 6 dans lequel les monomères ou mélanges de monomères sont polymérisés sous forme d'émulsion dans des solvants non miscibles.

8. Procédé selon la revendication 6 dans lequel le solvant comprend l'eau, un alcool aliphatique inférieur, l'acétone, méthyléthylcétone, les cétones cycliques, les carbonates d'éthyle et propyle, $\gamma$-butyrolactone, les glymes, les N-alkylpyrolidones, les tétraalkylsulfamides, le dichlorométhane, N-alkylimidazole, les hydrocarbures fluorés, et leurs mélanges.

9. Procédé selon la revendication 6 dans lequel un initiateur radicalaire de polymérisation est ajouté à la solution des monomères.

10. Procédé selon la revendication 9 dans lequel l'initiateur est activé thermiquement ou à l'aide de radiation actinique.

11. Procédé selon la revendication 10 dans lequel l'initiateur est un peroxyde ou un composé azo.

12. Procédé selon la revendication 6 dans lequel des additifs solides organiques ou inorganiques, sous forme de poudre de fibres, sont ajoutés à la solution avant la polymérisation pour améliorer les propriétés mécaniques des polymères, comme agent de formation de pores ou comme support de catalyseur.

13. Procédé selon la revendication 6 dans lequel au moins un monomère monofonctionnel est ajouté.

14. Procédé selon la revendication 13 dans lequel le monomère monofonctionnel est de formule $[T\text{-}SO_3]^- M^+$ ou $[T\text{-}SO_2\text{-}Y\text{-}SO_2\text{-}W]^- M^+$ dans lesquels T, Y et $M^+$ sont tels que définis à la revendication 1, Z représente F et W comprend un radical organique monovalent comprenant de 1 à 12 atomes de carbone, un radical organique monovalent comprenant de 1 à 12 atomes de carbone fluoré partiellement ou en totalité, un radical organique monovalent comprenant de 1 à 12 atomes de carbone substitué par un ou plusieurs oxa, aza, thia ou dioxathia et un radical organique monovalent comprenant de 1 à 12 atomes de carbone, fluoré partiellement ou en totalité et substitué par un ou plusieurs oxa, aza, thia, ou dioxathia fluoré partiellement ou en totalité, et optionnellement substitué par un ou plusieurs oxa, aza, thia, ou dioxathia.

15. Cellule électrochimique comprenant une membrane fabriquée avec un polymère selon l'une quelconque des revendications 3 à 5 ou avec un polymère obtenu à partir du procédé selon l'une quelconque des revendications 6 à 14, comme électrolyte solide.

16. Cellule électrochimique selon la revendication 15, **caractérisée en ce que** le polymère utilisé pour la préparation de ladite cellule a été formé de façon à être directement utilisable comme membrane.

17. Cellule selon la revendication 15 ou 16 comprenant une pile à combustible, un électrolyseur à l'eau, une pile chlore-soude, une pile électrochimique à recouvrement de sels ou d'acide, ou une pile produisant de l'ozone.

18. Cellule selon la revendication 15 ou 16 dans laquelle au moins une électrode est en contact avec la membrane.

19. Utilisation d'un polymère selon la revendication 3 dans un procédé d'électrolyse chlore-soude, comme séparateur dans une préparation électrochimique de composés organiques et inorganiques, comme séparateur entre une phase aqueuse et une phase organique, ou comme catalyseur pour les additions Diels-Alder, les réactions Friedel-Craft, les condensations aldol, la polymérisation cationique, les estérifications, et la formation d'acétals.

**Patentansprüche**

1. Bifunktionelles Monomer der allgemeinen Formel $[T\text{-}SO_2\text{-}Y\text{-}SO_2T']^- M^+$, worin:

- Y für N oder CQ, worin Q H, CN, F, $SO_2R^3$, gegebenenfalls substituiertes $C_{1-20}$-Alkyl, gegebenenfalls substituiertes $C_{1-20}$-Aryl oder gegebenenfalls substituiertes $C_{1-20}$-Alkylen bedeutet, steht, wobei der Substituent ein oder mehrere Halogenatome umfaßt und die Kette einen oder mehrere F-, $SO_2R$-, Aza-, Oxa-, Thia- oder Dioxathia-Substituenten enthält, wobei $R^3$ F, gegebenenfalls substituiertes $C_{1-20}$-Alkyl, gegebenenfalls substituiertes $C_{1-20}$-Aryl oder gegebenenfalls substituiertes $C_{1-20}$-Alkylen bedeutet, wobei der Substituent ein oder mehrere Halogenatome umfaßt;

- T und T' gleich oder verschieden sind und für:

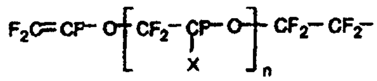

;

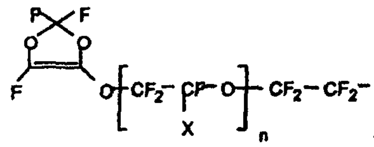

;

$CZ_2=CZ-$;

oder

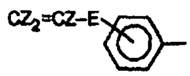

stehen, worin:

- X für ein Halogen oder $CF_3$ steht;
- n einen Wert zwischen 0 und 10 inklusive hat;
- E fehlt oder für 0, S oder $SO_2$ steht und
- Z für F oder H steht; und
- $M^+$ für ein anorganisches oder organisches Kation steht.

**2.** Monomer nach Anspruch 1 mit der Formel:

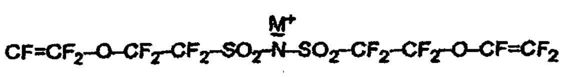

;

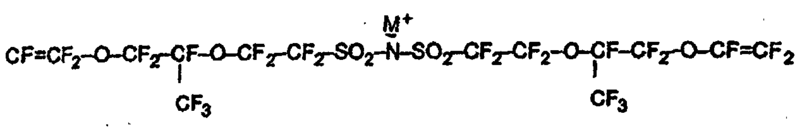

;

$[CF_2=CFSO_2-N-CFSO_2=CF_2]M^+$;

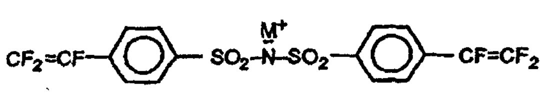

sowie Gemische davon.

3. Aus einem Monomer oder Monomerengemisch nach Anspruch 1 oder 2 erhaltenes Ionenaustauscher- und Festionenleiterpolymer.

4. Polymer nach Anspruch 3, bei dem das bifunktionelle Monomer mit mindestens einem monofunktionellen Monomer der Formel $[T\text{-}SO_3]M^+$ oder $[T\text{-}SO_2\text{-}Y\text{-}SO_2\text{-}W]M^+$, worin T, Y und $M^+$ die in Anspruch 1 angegebene Bedeutung besitzen, Z für F steht und W für einen monovalenten organischen Rest mit 1 bis 12 Kohlenstoffatomen, einen teil- oder perfluorierten monovalenten organischen Rest mit 1 bis 12 Kohlenstoffatomen, einen durch einen oder mehrere Oxa-, Aza-, Thia- oder Dioxathia-Substituenten substituierten monovalenten organischen Rest mit 1 bis 12 Kohlenstoffatomen oder einen teil- oder perfluorierten und durch einen oder mehrere Oxa-, Aza-, Thia- oder Dioxathia-Substituenten substituierten monovalenten organischen Rest mit 1 bis 12 Kohlenstoffatomen steht, copolymerisiert ist.

5. Polymer nach Anspruch 4, bei dem mindestens ein monofunktionelles Monomer der Formel:

$$CF_2{=}CF\text{-}SO_3^-M^+;$$

oder

$$CF_2{=}CF\text{-}O\text{-}CF_2CF_2SO_3^-M^+;$$

$$CF_2{=}CF\text{-}O\text{-}CF_2CF(CF_3)O\text{-}CF_2CF_2SO_3^-M^+;$$

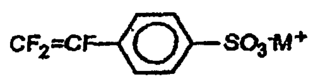

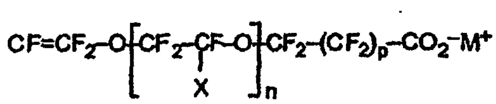

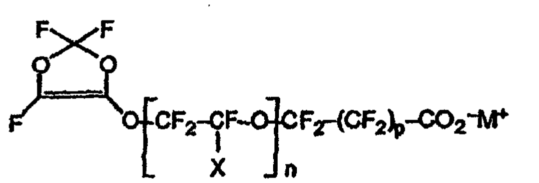

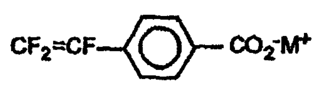

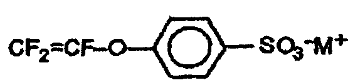

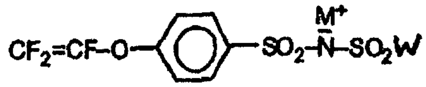

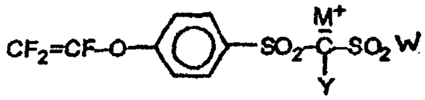

und

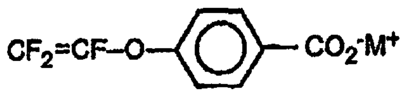

und Gemischen davon
entspricht, wobei M, X und n die in Anspruch 1 angegebene Bedeutung besitzen und W für einen monovalenten organischen Rest mit 1 bis 12 Kohlenstoffatomen, einen teil- oder perfluorierten monovalenten organischen Rest mit 1 bis 12 Kohlenstoffatomen, einen durch einen oder mehrere. Oxa-, Aza-, Thia- oder Dioxathia-Substituenten substituierten monovalenten organischen Rest mit 1 bis 12 Kohlenstoffatomen oder einen teil- oder perfluorierten und durch einen oder mehrere Oxa-, Aza-, Thia- oder Dioxathia-Substituenten substituierten monovalenten organischen Rest mit 1 bis 12 Kohlenstoffatomen steht.

**6.** Verfahren zur Herstellung eines Polymers aus Monomeren oder Monomerengemischen nach Anspruch 1, bei dem man die Monomere oder Monomerengemische in Lösung in einem Lösungsmittel polymerisiert, wobei das gebildete Polymer durch das Lösungsmittel homogen plastifiziert bleibt.

**7.** Verfahren nach Anspruch 6, bei dem man die Monomere oder Monomerengemische in Form einer Emulsion in nicht mischbaren Lösungsmitteln polymerisiert.

**8.** Verfahren nach Anspruch 6, bei dem man als Lösungsmittel Wasser, einen niederen aliphatischen Alkohol, Aceton, Methylethylketon, cyclische Ketone, Ethyl- und Propylcarbonat, $\gamma$-Butyrolacton, Glyme, N-Alkylpyrrolidone, Tetraalkylsulfamide, Dichlormethan, N-Alkylimidazole, fluorierte Kohlenwasserstoffe oder Gemische davon verwendet.

**9.** Verfahren nach Anspruch 6, bei dem man der Monomerenlösung einen radikalischen Polymerisationsinitiator zusetzt.

**10.** Verfahren nach Anspruch 9, bei dem der Initiator thermisch oder durch aktinische Strahlung aktiviert wird.

**11.** Verfahren nach Anspruch 10, bei dem man als Initiator ein Peroxid oder eine Azoverbindung einsetzt.

**12.** Verfahren nach Anspruch 6, bei dem man der Lösung vor der Polymerisation organische oder anorganische feste Additive in Pulver- oder Faserform zur Verbesserung der mechanischen Eigenschaften der Polymere, als Porenbildner oder als Katalysatorträger zusetzt.

**13.** Verfahren nach Anspruch 6, bei dem man mindestens ein monofunktionelles Monomer zusetzt.

**14.** Verfahren nach Anspruch 13, bei dem das monofunktionelle Monomer der Formel $[T\text{-}SO_3]^-M^+$ oder $[T\text{-}SO_2\text{-}Y\text{-}SO_2\text{-}W]^-M^+$ entspricht, worin T, Y und $M^+$ die in Anspruch 1 angegebene Bedeutung besitzen, Z für F steht und W für einen monovalenten organischen Rest mit 1 bis 12 Kohlenstoffatomen, einen teiloder perfluorierten monovalenten organischen Rest mit 1 bis 12 Kohlenstoffatomen, einen durch einen oder mehrere Oxa-, Aza-, Thia- oder Dioxathia-Substituenten substituierten monovalenten organischen Rest mit 1 bis 12 Kohlenstoffatomen oder einen teil- oder perfluorierten und durch einen oder mehrere Oxa-, Aza-, Thia- oder Dioxathia-Substituenten substituierten monovalenten organischen Rest mit 1 bis 12 Kohlenstoffatomen steht.

**15.** Elektrochemische Zelle, enthaltend eine mit einem Polymer nach einem der Ansprüche 3 bis 5 oder einem nach einem Verfahren nach einem der Ansprüche 6 bis 14 erhaltenen Polymer als Festelektrolyt hergestellte Membran.

**16.** Elektrochemische Zelle nach Anspruch 15, **dadurch gekennzeichnet, daß** das zur Herstellung der Zelle verwendete Polymer in einer solchen Form erhalten wird, daß es direkt als Membran verwendet werden kann.

**17.** Zelle nach Anspruch 15 oder 16, bei der es sich um eine Brennstoffzelle, eine Wasserelektrolysezelle, eine Chloralkali-Zelle, eine elektrochemische Zelle zur Rückgewinnung von Salzen oder Säure oder eine Ozon produzierende Zelle handelt.

**18.** Zelle nach Anspruch 15 oder 16, bei der mindestens eine Elektrode mit der Membran in Berührung steht.

**19.** Verwendung eines Polymers nach Anspruch 3 bei einem Chloralkali-Elektrolyseverfahren, als Separator bei der elektrochemischen Herstellung von organischen und anorganischen Verbindungen, als Separator zwischen einer wäßrigen Phase und einer organischen Phase oder als Katalysator für Diels-Alder-Additionen, Friedel-Crafts-Reaktionen, Aldolkondensationen, kationische Polymerisationen, Veresterungen und die Bildung von Acetalen.

## Claims

**1.** Bifunctional monomer of general formula $[T\text{-}SO_2\text{-}Y\text{-}SO_2T']^{-}M^{+}$, in which:

- Y comprises N or CQ in which Q comprises H, CN, F, $SO_2R^3$, substituted or unsubstituted $C_{1\text{-}20}$ alkyl; substituted or unsubstituted $C_{1\text{-}20}$ aryl; substituted or unsubstituted $C_{1\text{-}20}$ alkylene, in which radicals the substituent comprises one or more halogens, and in which radicals the chain comprises one or more F, $SO_2R$, aza, oxa, thia or dioxathia substitutents, with $R^3$ comprising F, substituted or unsubstituted $C_{1\text{-}20}$ alkyl; substituted or unsubstituted $C_{1\text{-}20}$ aryl; substituted or unsubstituted $C_{1\text{-}20}$ alkylene, in which radicals the substituent comprises one or more halogens;
- T and T' are identical or different and comprise:

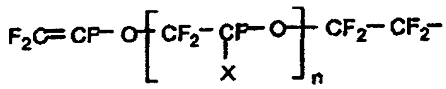
;

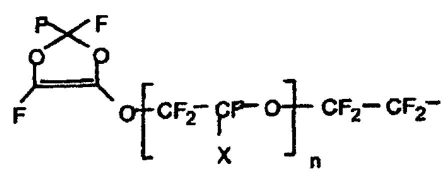
;

$CZ_2$=CZ-;and

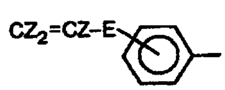

in which:

- X comprises a halogen or $CF_3$;
- N varies between 0 and 10 inclusive:
- E is absent, O, S, $SO_2$; and
- Z is F or H; and
- $M^+$ comprises an inorganic or organic cation.

**2.** Monomer according to Claim 1, of formula:

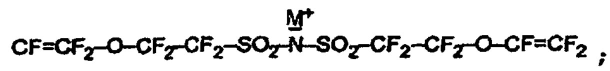

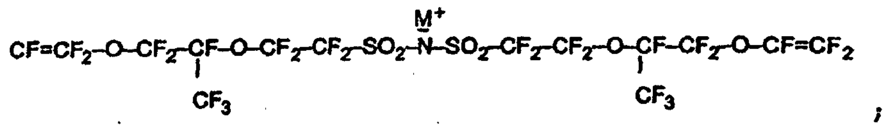

$$[CF_2=CFSO_2\text{-}N\text{-}CFSO_2=CF_2]M^+;$$

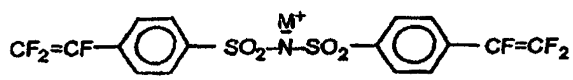

and their mixtures.

**3.** Ion-exchange and solid ion-conducting polymer obtained from a monomer or a mixture of monomers according to Claim 1 or 2.

**4.** Polymer according to Claim 3, in which the bifunctional monomer is copolymerized with at least one monofunctional monomer of formula $[T\text{-}SO_3]^-M^+$ or $[T\text{-}SO_2\text{-}Y\text{-}SO_2\text{-}W]^-M^+$ in which T, Y and $M^+$ are as defined in Claim 1, Z represents F and W comprises a monovalent organic radical comprising from 1 to 12 carbon atoms, a partially or completely fluorinated monovalent organic radical comprising from 1 to 12 carbon atoms, a monovalent organic radical comprising from 1 to 12 carbon atoms which is substituted by one or more oxa, aza, thia or dioxathia and a partially or completely fluorinated monovalent organic radical comprising from 1 to 12 carbon atoms which is substituted by one or more oxa, aza, thia or dioxathia.

**5.** Polymer according to Claim 4, in which at least one monofunctional monomer is of formula:

$$CF_2=CF\text{-}SO_3^-M^+;$$

or

$$CF_2=CF\text{-}O\text{-}CF_2CF_2SO_3^-M^+;$$

$$CF_2=CF\text{-}O\text{-}CF_2CF(CF_3)O\text{-}CF_2CF_2SO_3^-M^+;$$

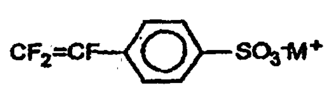

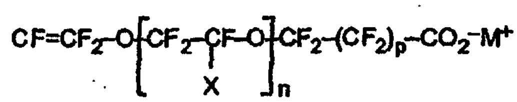

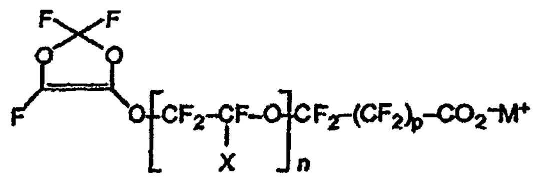

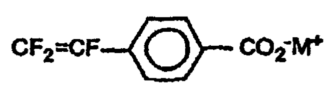

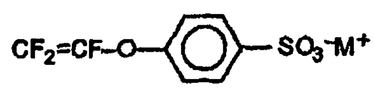

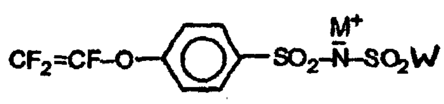

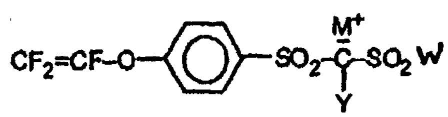

and

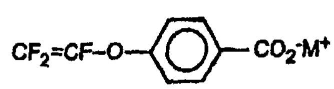

and their mixtures, and in which monomers M, X and n are as defined in Claim 1 and W comprises a monovalent organic radical comprising from 1 to 12 carbon atoms, a partially or completely fluorinated monovalent organic radical comprising from 1 to 12 carbon atoms, a monovalent organic radical comprising from 1 to 12 carbon atoms which is substituted by one or more oxa, aza, thia or dioxathia and a partially or completely fluorinated monovalent organic radical comprising from 1 to 12 carbon atoms which is substituted by one or more oxa, aza, thia or dioxathia.

**6.** Process for the preparation of a polymer from monomers or mixtures of monomers according to Claim 1, in which the monomers or mixtures of monomers are polymerized in solution in a solvent, the polymer formed remaining homogeneously plasticized by the solvent.

**7.** Process according to Claim 6, in which the monomers or mixtures of monomers are polymerized in the form of an emulsion in immiscible solvents.

**8.** Process according to Claim 6, in which the solvent comprises water, a lower aliphatic alcohol, acetone, methyl ethyl ketone, cyclic ketones, ethyl and propyl carbonates, $\gamma$-butyrolactone, glymes, N-alkylpyrrolidones, tetraalkylsulphamides, dichloromethane, N-alkylimidazoles, fluorinated hydrocarbons, and their mixtures.

**9.** Process according to Claim 6, in which a polymerization free-radical initiator is added to the solution of the monomers.

10. Process according to Claim 9, in which the initiator is activated thermally or using actinic radiation.

11. Process according to Claim 10, in which the initiator is a peroxide or an azo compound.

12. Process according to Claim 6, in which solid organic or inorganic additives in the form of powder or fibres are added to the solution before the polymerization in order to improve the mechanical properties of the polymers, as pore-forming agent or as catalyst support.

13. Process according to Claim 6, in which at least one monofunctional monomer is added.

14. Process according to Claim 13, in which the monofunctional monomer is of formula $[T\text{-}SO_3]^-M^+$ or $[T\text{-}SO_2\text{-}Y\text{-}SO_2\text{-}W]^-M^+$ in which T, Y and $M^+$ are as defined in Claim 1, Z represents F and W comprises a monovalent organic radical comprising from 1 to 12 carbon atoms, a partially or completely fluorinated monovalent organic radical comprising from 1 to 12 carbon atoms, a monovalent organic radical comprising from 1 to 12 carbon atoms which is substituted by one or more oxa, aza, thia or dioxathia and a partially or completely fluorinated monovalent organic radical comprising from 1 to 12 carbon atoms which is substituted by one or more oxa, aza, thia or dioxathia.

15. Electrochemical cell, comprising a membrane manufactured with a polymer according to any one of Claims 3 to 5 or with a polymer obtained from the process according to any one of Claims 6 to 14, as solid electrolyte.

16. Electrochemical cell according to Claim 15, **characterized in that** the polymer used for the preparation of the said cell was formed so as to be directly useable as membrane.

17. Cell according to Claim 15 or 16, comprising a fuel cell, a water electrolyser, a chlorine-sodium hydroxide cell, an electrochemical cell with recovery of salts or of acid, or an ozone-producing cell.

18. Cell according to Claim 15 or 16, in which at least one electrode is in contact with the membrane.

19. Use of a polymer according to Claim 3 in a chlorine-sodium hydroxide electrolysis process, as separator in an electrochemical preparation of organic and inorganic compounds, as separator between an aqueous phase and an organic phase, or as catalyst for Diels-Alder additions, Friedel-Craft reactions, aldol condensations, cationic polymerization, esterifications and the formation of acetals.